# DEMANDE DE BREVET EUROPEEN

(11) **EP 0 649 646 A1**
(43) Date de publication de la demande: **26.04.1995**
(21) Numéro de dépôt: 93490019.2
(22) Date de dépôt: 20.10.1993
(51) Int. Cl.: A61K 7/155

(54) **Préparation épilatoire, procédé de réalisation d'une telle préparation et procédé d'épilation utilisant ladite préparation épilatoire**

(71) Demandeur: Le Brun, Aicha, F-30390 Aramon (FR)
(72) Inventeur: Le Brun, Aicha, F-30390 Aramon (FR)
(74) Mandataire: Lepage, Jean-Pierre

(57) **Abrégé**

L'invention est relative à une préparation épilatoire, à un procédé de réalisation d'une telle préparation ainsi qu'à un procédé d'épilation utilisant ladite préparation épilatoire.

Selon l'invention, la préparation épilatoire, destinée à être appliquée sur le corps à froid pour l'arrachement des poils, se présente sous la forme d'une mixture cuite de tout sucre et de toute forme de vinaigre.

Plus précisément, dans un mode de réalisation avantageux, on mélange en volume deux mesures de sucre blanc en poudre et une mesure de vinaigre d'alcool blanc, puis on porte le mélange à ébullition et on malaxe le mélange jusqu'à obtention d'une pâte prête à l'emploi.

Elle trouvera son application dans le domaine des soins du corps.

## Description

L'invention est relative à une préparation épilatoire, et à un procédé de réalisation d'une telle préparation, ainsi qu'à un procédé d'épilation utilisant ladite préparation épilatoire.

Elle trouvera son application dans le domaine des compositions pour les soins du corps.

Dans ce domaine, on connaît des compositions épilatoires constituées à base de cire, naturelle ou artificielle, que l'on dépose à chaud sur la zone à épiler, pour noyer les poils dans la couche de cire, que l'on enlève ensuite après refroidissement.

De tels produits donnent de bons résultats sur le plan de l'arrachement des poils mais ne sont pas sans poser certains problèmes de réaction de la peau.

En effet, il est important dans ce cas de maîtriser la chaleur à laquelle la cire est déposée pour éviter de brûler l'utilisateur. En outre, certains présentent des allergies aux différents produits, ce qui se traduit par des rougeurs épidermiques disgracieuses. Par ailleurs, certaines cires sont difficiles à nettoyer de par leur composition, ce qui nécessite de prévoir une protection lors de l'utilisation.

Pour éviter ces inconvénients et éviter d'avoir recours à des cires épilatoires, certains constructeurs ont mis au point des appareils à épiler mécaniques, actionnés par un moteur électrique. La zone de travail est constituée par une spirale métallique qui se déforme ou encore par des disques plats qui, alternativement, se rapprochent et s'écartent les uns des autres pour emprisonner les poils et les arracher.

Ces dispositifs connaissent un certain succès mais ne sont pas néanmoins d'une utilisation universelle. En outre, les poils doivent avoir déjà une certaine importance et rigidité pour être pris dans la spirale ou entre les disques.

Le but de la présente invention est de proposer une préparation épilatoire, destinée à être appliquée sur le corps à froid pour l'arrachement des poils, qui permette de pallier les inconvénients précités.

En particulier, la préparation de la présente invention permet un travail facile, évite les désagréments des brûlures et des allergies. Elle peut permettre l'épilation de toutes zones sensibles du corps.

En outre, la préparation épilatoire de la présente invention est un produit non toxique, qui se nettoie facilement, à l'eau notamment.

Un autre but de la présente invention est de proposer un procédé de réalisation d'une telle préparation épilatoire, qui soit facile à mettre en oeuvre et qui ne nécessite pas d'installation particulière de fabrication.

En outre, son coût de revient est remarquable ce qui confère à la préparation un excellent rapport qualité/prix.

D'autres buts et avantages de la présente invention apparaîtront au cours de la description qui va suivre qui n'est cependant donnée qu'à titre indicatif et qui n'a pas pour but de la limiter.

Selon l'invention, la préparation épilatoire, destinée à être appliquée sur le corps à froid pour l'arrachement des poils, est caractérisée par le fait qu'elle se présente sous la forme d'une mixture cuite de tout sucre et de toute forme de vinaigre.

Selon le procédé de réalisation de la préparation épilatoire de la présente invention, on mélange du sucre et du vinaigre, puis on porte le mélange à ébullition et on malaxe le mélange jusqu'à obtention d'une pâte prête à l'emploi.

Selon le procédé d'épilation utilisant la préparation épilatoire de la présente invention, on applique à froid une couche de la préparation épilatoire sur la zone du corps à épiler pour emprisonner les poils dans ladite couche et on enlève rapidement la pâte dans le sens du poil.

La présente invention sera mieux comprise à la lecture de la description suivante.

La présente invention vise une préparation épilatoire, destinée à être appliquée sur le corps à froid pour l'arrachement des poils, un procédé de réalisation d'une telle préparation ainsi qu'un procédé d'épilation utilisant ladite préparation épilatoire.

Selon la présente invention, la préparation est constituée par un mélange de sucre et de vinaigre.

D'une façon générale, la préparation peut être obtenue à partir de tout sucre et toute forme de vinaigre. Par exemple, on a obtenu de bons résultats en utilisant du sucre blanc et du vinaigre d'alcool blanc.

Cette mixture est ensuite cuite avant son utilisation pour arriver à l'obtention d'une pâte prête à l'emploi.

En particulier, la préparation de la présente invention comporte en volume de une à trois mesures de sucre blanc en poudre pour une mesure de vinaigre d'alcool blanc.

A titre d'exemple, pour une pâte facile à mettre en oeuvre et donnant de bons résultats au niveau de l'arrachement des poils, on a utilisé deux mesures de sucre blanc en poudre, c'est-à-dire environ 110 grammes de sucre blanc pour une mesure de vinaigre d'alcool blanc, c'est-à-dire environ 80 millilitres de vinaigre d'alcool blanc.

Pour préparer cette composition épilatoire, on mélange donc les composants dans les quantités rappelées ci-dessus puis on porte le mélange à ébullition, on maintient le mélange à ébullition pendant un temps donné, ce temps de cuisson étant sensiblement proportionnel au nombre de mesures utilisées.

A titre d'exemple, pour un mélange en volume de deux mesures de sucre blanc en poudre et une mesure de vinaigre d'alcool blanc, on maintiendra l'ébullition pendant environ 7 minutes en remuant de temps en temps.

Dans le cas ou l'on réalise un mélange de 330 grammes de sucre blanc, c est-à-dire six mesures, et de 240 millilitres de vinaigre d'alcool blanc, soit trois mesures, il faut alors maintenir l'ébullition pendant environ 20 minutes.

Après cuisson, c'est-à-dire après avoir maintenu l'ébullition pendant un temps donné, ce en remuant de temps en temps, on laisse refroidir la mixture cuite et dès que la pâte est tiède, on malaxe le mélange jusqu'à obtention d'une pâte prête à l'emploi, c'est-à-dire une pâte qui va être facile à appliquer sur le corps et qui va conférer une bonne adhérence des poils pour faciliter l'arrachage.

Cela étant, selon le procédé d'épilation utilisant la préparation épilatoire de la présente invention, on applique à froid une couche de la préparation épilatoire ainsi obtenue sur la zone du corps à épiler pour emprisonner les poils dans ladite couche.

Il est à noter que pour malaxer la pâte et l'appliquer sur le corps, l'opérateur s'humidifiera légèrement les mains pour faciliter le travail de la pâte.

Ensuite, dès que la pâte a été appliquée sur le corps, on l'enlève rapidement dans le sens du poil en arrachant ceux-ci. Cependant, on pourra également opérer à contre sens du poil.

Contrairement aux cires traditionnelles, il n'est pas nécessaire d'attendre que la pâte durcisse pour l'enlever car, grâce à sa structure et sa capacité de faire adhérer les poils, la pâte peut être enlevée sans délai d'attente.

Grâce à la présente invention, on obtient donc une préparation épilatoire qui n'est pas toxique et qui n'engendre pas de problème d'allergie, notamment pour les personnes atteintes de varices. En outre, c'est un produit qui se travaille bien, qui ne colle pas et qui s'enlève facilement notamment à l'eau tiède.

Par ailleurs, il est à noter que la conservation de cette pâte dépend de son mode de conditionnement et peut aller de deux mois à l'air libre, à plusieurs mois au réfrigérateur et jusque plusieurs années au congélateur. Elle est en outre biodégradable et sans danger en cas d'ingestion.

Selon la façon dont la pâte sera conditionnée, par exemple en pot, tube ou flacon, on pourra être amené à introduire un produit évitant à la pâte de durcir trop rapidement.

Pour des raisons d'ordre esthétique, la pâte peut également être parfumée et/ou colorée.

Naturellement, d'autres mises en oeuvre de la présente invention, à la portée de l'Homme de l'Art, auraient pu être envisagées sans pour autant sortir du cadre de celle-ci.

## Revendications

1. Préparation épilatoire, destinée à être appliquée sur le corps à froid pour l'arrachement des poils, se présentant sous la forme d'une mixture cuite de tout sucre et de toute forme de vinaigre, caractérisée par le fait qu'elle comporte en volume de une à trois mesures de sucre blanc en poudre pour une mesure de vinaigre d'alcool blanc.

2. Préparation épilatoire, selon la revendication 1, caractérisée par le fait qu'elle comporte pour 110 grammes de sucre blanc, 80 millilitres de vinaigre d'alcool blanc.

3. Procédé de réalisation d'une préparation épilatoire, selon la revendication 1, caractérisé par le fait que :
- on mélange en volume une à trois mesures de sucre blanc en poudre pour une mesure de vinaigre d'alcool blanc,
- on porte le mélange à ébullition et on le maintient pendant 7 minutes,
- on laisse refroidir la mixture cuite et on la malaxe lorsqu'elle est tiède.

4. Procédé de réalisation d'une préparation épilatoire selon la revendication 3, caractérisé par le fait que le temps de cuisson est sensiblement proportionnel au nombre de mesures.

5. Procédé d'épilation utilisant la préparation épilatoire selon la revendication 1, caractérisé par le fait que l'on applique à froid une couche de la préparation épilatoire sur la zone du corps à épiler pour emprisonner les poils dans ladite couche et on enlève rapidement la pâte dans le sens du poil.
